(19) Europäisches Patentamt
European Patent Office
Office européen des brevets

(11) EP 0 698 613 B1

(12) EUROPEAN PATENT SPECIFICATION

(45) Date of publication and mention of the grant of the patent:
**24.03.1999 Bulletin 1999/12**

(51) Int Cl.[6]: **C07K 7/08**

(21) Application number: **95111532.8**

(22) Date of filing: **21.07.1995**

# (54) Endothelin-antagonizing peptide

Endothelin-antagonisierendes Peptid

Peptide qui est un antagoniste de l'endothelin

(84) Designated Contracting States:
**AT BE CH DE DK ES FR GB GR IE IT LI LU MC NL PT SE**

(30) Priority: **25.07.1994 JP 172689/94**

(43) Date of publication of application:
**28.02.1996 Bulletin 1996/09**

(73) Proprietor: **KYOWA HAKKO KOGYO CO., Ltd.**
**Chiyoda-ku, Tokyo 100 (JP)**

(72) Inventors:
- **Yamasaki, Motoo**
  **Machida-shi, Tokyo 194 (JP)**
- **Shibata, Kenji**
  **Kawasaki-shi, Kanagawa 215 (JP)**
- **Tanaka, Takeo**
  **Machida-shi, Tokyo 194 (JP)**
- **Matsuda, Yuzuru**
  **Koganei-shi, Tokyo 184 (JP)**

(74) Representative: **VOSSIUS & PARTNER**
**Siebertstrasse 4**
**81675 München (DE)**

(56) References cited:
**EP-A- 0 487 410** **WO-A-93/13218**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

The present invention relates to a peptide which has endothelin-antagonizing activity. The peptide of the present invention has excellent endothelin-antagonizing activity, and is therefore useful for the treatment of hypertension, asthma, cerebral apoplexy, angina pectoris, acute renal failure, cardiac infarction, cerebral vasospasm, etc.

Endothelin is a cyclic peptide which possesses a strong, long-lasting vasoconstricting effect, and is thought to be one of the substances responsible for hypertension, asthma, cerebral apoplexy, angina pectoris, acute renal failure, cardiac infarction, and cerebral vasospasm. Consequently, a substance which antagonizes endothelin and inhibits its effects is expected to be useful for the treatment and prevention of these diseases.

It is known that the peptide represented by the following formula (A):

Gly–Asn–Trp–His–Gly–Thr–Ala–Pro–Asp–Trp–
Phe–Phe–Asn–Tyr–Tyr–Z (A)

wherein Z represents a naturally-occurring amino acid residue, exhibits endothelin antagonism (WO93/13218). However, there is no disclosure about peptides having a non-naturally-occurring amino acid residue.

According to the present invention, there is provided a peptide compound represented by the following formula (I):

Gly–Asn–Trp–His–Gly–Thr–Ala–Pro–Asp–Trp–
Phe–Phe–Asn–Tyr–Tyr–X (I)

wherein X represents

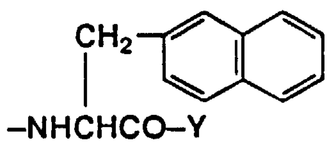

or

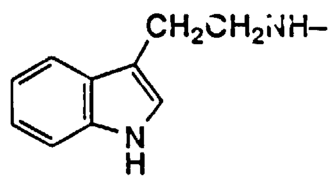

(wherein Y represents hydroxy, lower alkoxy, benzyloxy, benzhydryloxy, or amino), or a pharmaceutically acceptable salt thereof.

The peptide compound represented by the above formula (I) is hereinafter referred to as Compound (I), and the same applies to the compounds of other formula numbers.

In the definitions for the above formula (I), the alkyl moiety of lower alkoxy means a straight-chain or branched alkyl group having 1 to 6 carbon atoms such as methyl, ethyl, propyl, isopropyl, butyl, isobutyl, secbutyl, tert-butyl, pentyl, neopentyl, hexyl, and isohexyl.

The pharmaceutically acceptable salts of Compound (I) include pharmaceutically acceptable acid addition salts, metal salts, and organic base addition salts.

Examples of the pharmaceutically acceptable acid addition salts are inorganic acid addition salts such as hydrochloride, sulfate, and phosphate, and organic acid addition salts such as acetate, maleate, fumarate, tartrate, and citrate. Examples of the pharmaceutically acceptable metal salts are alkali metal salts such as the sodium salt and potassium salt, alkaline earth metal salts such as the magnesium salt and calcium salt, aluminum salts, and zinc salts. Examples of the pharmaceutically acceptable organic base addition salts are salts with primary amines such as methylamine, ethylamine, and aniline, secondary amines such as dimethylamine, diethylamine, pyrrolidine, piperidine, morpholine, and piperazine, and tertiary amines such as trimethylamine, triethylamine, N,N-dimethylaniline, and pyridine, and ammonium salts.

The present invention is described in detail below.

The abbreviations for the amino acids and their protective groups used in this specification follow the recommendations of the IUPAC-IUB Joint Commission relating to biochemical nomenclature [Eur. J. Biochem., 138, 9 (1984)].

Unless otherwise provided, the following abbreviations indicate the corresponding amino acids and protective groups.

Gly: glycine
Ala: L-alanine
Thr: L-threonine
Pro: L-proline
Asp: L-aspartic acid
Asn: L-asparagine
Asx: L-aspartic acid or L-asparagine
His: L-histidine
Phe: L-phenylalanine
Tyr: L-tyrosine
Trp: L-tryptophan
Nal: L-β-(2-naphthyl)alanine

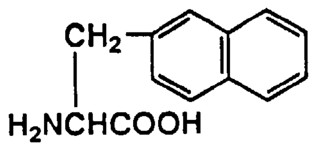

(Nal)

Trn: 2-(3-indolyl)ethylamine

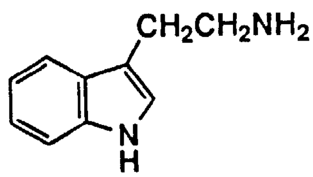

(Trn)

Fmoc: 9-fluorenylmethyloxycarbonyl

The following abbreviation indicates the corresponding side-chain-protected amino acid.
H-Nal-OBzl($NO_2$): L-β-(2-naphthyl)alanine 4-nitrobenzyl ester.

The following abbreviations indicate the corresponding reaction solvents and reagents.

PyBOP : benzotriazol-1-yloxytripyrrolidinophosphonium hexafluorophosphate
HOBt: N-hydroxybenzotriazole
NMM: N-methylmorpholine
DMF: N,N-dimethylformamide
TFA: trifluoroacetic acid

The processes for producing Compound (I) of the present invention are described below.

Compound (I) can be prepared according to the following reaction scheme.

Gly–Asn–Trp–His–Gly–Thr–Ala–Pro–Asp–Trp–
Phe–Phe–Asn–Tyr–Tyr–Trp–OH

(RES–701–1)

Gly–Asn–Trp–His–Gly–Thr–Ala–Pro–Asp–Trp–
Phe–Phe–Asn–Tyr–Tyr–OH (B)

H–X (II)

Gly–Asn–Trp–His–Gly–Thr–Ala–Pro–Asp–Trp–
Phe–Phe–Asn–Tyr–Tyr–X (I)

(In the formulae, X has the same meaning as defined above.)

Compound (B) can be obtained by treating RES-701-1 (WO93/13218) with a protease such as carboxypeptidase. Then, Compound (I) can be obtained by condensing Compound (3) with Compound (II) or appropriately protected Compound (II) using a condensing agent (for example, PyBOP/HOBt/NMM) and then, if necessary, deprotecting the product according to the conventional method (for example, "Fundamentals and Experiments in Peptide Synthesis", Nobuo Izumiya et al., Maruzen).

The thus obtained Compound (I) may be purified by high pressure liquid chromatography (referred to as HPLC hereinafter) using a C-4, C-8, or C-18 reverse phase silica gel column, column chromatography such as partition, adsorption resin, silica gel, chemically modified silica gel, reverse phase silica gel, alumina, diatomaceous earth, magnesium silicate, ion-exchange resin, and gel filtration, or thin layer chromatography.

A conventional method is used to obtain a pharmaceutically acceptable salt of Compound (I). That is, an acid addition salt or an organic base addition salt of Compound (I) can be obtained by dissolving Compound (I) in an aqueous solution of a corresponding acid or organic base, and freeze-drying the resultant solution. In addition, a metal salt of Compound (I) can be obtained by dissolving Compound (I) in an aqueous solution containing a corresponding metal ion, and purifying the solution by gel filtration or HPLC.

Examples of Compound (I) are shown in Table 1.

Table 1

| Compd. No. | Structure |
|---|---|
| (I–1) | Gly–Asn–Trp–His–Gly–Thr–Ala–Pro–Asp–Trp–Phe–Phe–Asn–Tyr–Tyr–Trn |
| (I–2) | Gly–Asn–Trp–His–Gly–Thr–Ala–Pro–Asp–Trp–Phe–Phe–Asn–Tyr–Tyr–Nal–OH |

The pharmacological activities of Compound (I) are described below by test examples.

Test Example 1: Endothelin Receptor-Antagonizing Activity

Bovine cerebellum tissue was homogenized at 4°C by using POLYTRON (type PT10/35, manufactured by Kinematica Gmbh) in a buffer solution A (1mM $NaHCO_3$, 5 mM ethylenediaminetetraacetic acid, 5 µg/ml leupeptin, 5 µg/ml pepstatin A, 40 µM phenylmethylsulfonyl fluoride, pH 8.3).

The obtained suspension was centrifuged for 10 minutes at 8,000 G and 4°C, and the resulting supernatant was centrifuged for 60 minutes at 40,000 G and 4°C to give pellets. The obtained pellets were suspended in a buffer solution A and again centrifuged for 60 minutes at 40,000 G and 4°C. The resulting solid substance was prepared as a suspension containing 2 mg/ml of protein, and the suspension was used as a membrane fraction liquid. A membrane fraction solution was prepared by adding 7 µl of the membrane fraction liquid per 1 ml of a buffer solution B (50 mM Tris-HCl, 1 mM ethylenediaminetetraacetic acid, 0.2% bovine serum albumin, pH 7.6). $^{125}I$-Endothelin-1 (approx. 30,000 cpm) was added to the membrane fraction solution containing unlabelled endothelin-1 (final concentration 100 nM) or any one of the test compounds, or containing neither of them. These mixtures were incubated at 25°C for 2 hours, and then filtered with a GF/B glass filter (produced by Whatman Co.). After washing the filter with a buffer solution C (50 mM Tris-HCl, 1 mM ethylenediaminetetraacetic acid, pH 7.6), the radioactivity on the glass filter was measured to determine the amount of the receptor and the non-specific bound $^{125}I$-endothelin. The inhibition rate against endothelin receptor binding was calculated according to the following equation:

$$\text{Inhibition rate (\%)} = (C - A/C - B) :< 100$$

A: Radioactivity in the presence of one of the test compounds
B: Radioactivity in the presence of unlabelled endothelin-1
C: Radioactivity in the absence of both of the test compound and unlabelled endothelin-1

The results are shown in Table 2.

Table 2

| Compound No. | $IC_{50}$ (nM) |
|---|---|
| (I-1) | 50 |
| (I-2) | 6.3 |
| $IC_{50}$: Concentration causing 50 % inhibition of endothelin-1 binding | |

Test Example 2: Endothelin Receptor-Antagonizing Activity

The inhibition rate against endothelin receptor binding was calculated by the same method as in Test Example 1, except for using bovine lung tissue instead of the bovine cerebellum tissue used in Test Example 1, and adding RES-701-1 to the membrane fraction solution at a concentration of 1.5 µg/ml,

Table 3

| Compound No. | $IC_{50}$ (nM) |
|---|---|
| (I-1) | 300 |
| (I-2) | >440 |
| $IC_{50}$: Concentration causing 50 % inhibition of endothelin-1 binding | |

Examples and Reference Example of the present invention are described below.

Example 1: Synthesis of Compound (I-1)

0.1 ml of a DMF solution containing 0.84 mg of PyBOP, 0.1 ml of a DMF solution containing 0.22 mg of HOBt, and 0.1 ml of a DMF solution containing 0.3 µl of NMM were added to 0.5 ml of a DMF solution containing 1 mg of Compound (B) obtained in Reference Example 1, followed by standing at 0°C for 10 minutes. Then, 0.1 ml of a DMF solution containing 0.53 mg of H-Trn·HCl and 0.3 µl of NMM was added thereto, and the mixture was stirred for 16

hours at 4°C. 0.1 ml of 2M acetic acid was added thereto, and the crude product was subjected to HPLC using a reverse phase column (CAPCELL PACK C18, 250 mm x 30 mm I.D., manufactured by Shiseido). The elution was carried out with a linear concentration gradient pattern using a 0 to 90% aqueous acetonitrile solution containing 0.1% TFA, and upon detection at 220 nm, to give fractions containing the entitled Compound (I-1). These fractions were lyophilized to give 0.1 mg of Compound (I-1).

| | |
|---|---|
| MS analysis [FABMS]: | 1998 (M + H) |
| Amino acid analysis: | Found (Theoretical) Gly 2.2 (2), Ala 1.0 (1), Asx 2.0 (3), His 1.0 (1), Pro 1.1 (1), Thr 1.1 (1), Tyr 1.9 (2), Phe 1.9 (2), Trp and Trn not analyzed |

Example 2: Synthesis of Compound (I-2)

Step 1: Synthesis of H-Nal-OBzl($NO_2$)

43.75 mg of Fmoc-Nal-OH was dissolved in 1 ml of DMF, and 16.8 mg of sodium bicarbonate and 108 mg of 4-nitrobenzyl bromide were added thereto, followed by stirring for 17 hours at room temperature. 30 ml of ethyl acetate and 70 ml of water were added thereto followed by shaking, and the ethyl acetate layer was recovered and dried over sodium sulfate. The mixture was filtered, and the solvent was distilled off from the filtrate. The residue was subjected to silica gel column chromatography (Kieselgel 60, manufactured by Merck Co., eluent: ethyl acetate/hexane) to give Fmoc-Nal-OBzl($NO_2$) as a white powder. The obtained powder was dissolved in a 20% DMF solution of piperidine followed by standing at room temperature for 20 minutes. The solvent was distilled off under reduced pressure, and the residue was purified by HPLC in the same manner as in Example 1 to give 33.6 mg of H-Nal-OBzl($NO_2$).

| | |
|---|---|
| MS analysis [FABMS] : | 351 (M + H) |

Step 2: Synthesis of Compound (I-2)

10μl of a DMF solution containing 84 μg of PyBOP, 10μl of a DMF solution containing 22 μg of HOBt, and 10 μl of a DMF solution containing 0.03 μl of NMM were added to 55 μl of a DMF solution containing 100 μg of Compound (B) obtained in Reference Example 1, followed by standing at 0°C for 10 minutes. Then, 10 μl of a DMF solution containing 125 μg of H-Nal-OBzl($NO_2$) obtained in Step 1 and 0.03 μl of NMM was added thereto, and the mixture was stirred for 50 hours at 4°C. 100 μl of 90% acetic acid was added thereto, and then about 5 mg of zinc powder was added thereto under ice cooling, followed by standing for 10 minutes. The temperature of the mixture was brought back to room temperature followed by stirring for one hour. After centrifugation, the resulting supernatant was purified by HPLC in the same manner as in Example 1 to give 14 μg of Compound (I-2).

| | |
|---|---|
| MS analysis [FABMS]: | 2053 (M + H) |
| Amino acid analysis: | Found (Theoretical) Gly 2.3 (2), Ala 1.0 (1), Asx 2.0 (3), His 1.0 (1), Pro 1.1 (1), Thr 1.0 (1), Tyr 1.9 (2), Phe 1.9 (2), Trp and Nal not analyzed |

Reference Example 1: Synthesis of Compound (B)

7.1 mg of RES-701-1 was dissolved in 2.84 ml of methanol, and 25.56 ml of a 0.1M Tris-HCl buffer solution (pH 8.0) and then about 0.3 mg of carboxypeptidase A (C-9762, produced by Sigma Co.) were added thereto, followed by stirring at 37°C for one night. The reaction mixture was acidified by addition of an appropriate amount of hydrochloric acid, and the crude product was subjected to reverse phase HPLC equipped with a NUCLEOSIL 5C18 column (250 x 20 mm I.D., manufactured by Chemco Inc.) with a linear concentration gradient pattern wherein an increase in the concentration of acetonitrile in the 0.1% TFA solution from 0% to 50% was effected in 30 minutes, at a flow rate of 10 ml/min, to give 3.5 mg of Compound (B).

| | |
|---|---|
| MS analysis [FABMS]: | 1858 (M + H) |
| Amino acid analysis: | Found (Theoretical) Gly 2.2 (2), Ala 1.0 (1), Asx 2.6 (3), His 0.9 (1), Pro 1.1 (1), Thr 1.0 (1), Tyr 1.8 (2), Phe 1.9 (2), Trp not analyzed |

## Claims

**1.** A peptide compound represented by the following formula (I):

Gly–Asn–Trp–His–Gly–Thr–Ala–Pro–Asp–Trp–
Phe–Phe–Asn–Tyr–Tyr–X (I)

wherein X represents

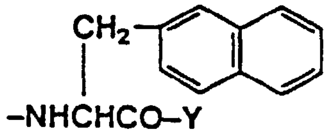

or

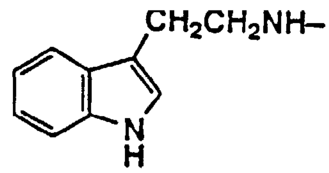

(wherein Y represents hydroxy, lower alkoxy, benzyloxy, benzhydryloxy, or amino), or a pharmaceutically acceptable salt thereof.

2. A peptide compound according to Claim 1 wherein X is

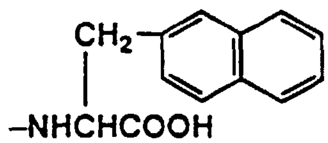

, or a pharmaceutically acceptable salt thereof.

3. A peptide compound according to Claim 1 wherein X is

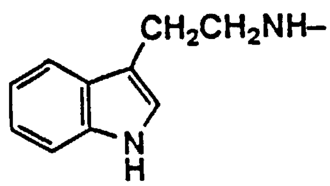

, or a pharmaceutically acceptable salt thereof.

4. The peptide of any one of claims 1 to 3 for use as endothelin receptor-antagonizing agent.

5. Use of the peptide of any one of claims 1 to 3 for the preparation of a pharmaceutical composition for the treatment of hypertension, asthma, cerebral apoplexy, angina pectoris, acute renal failure, cardiac infraction or cerebral vasospasm.

**Patentansprüche**

1. Peptidverbindung mit der folgenden Formel (1):

Gly–Asn–Trp–His–Gly–Thr–Ala–Pro–Asp–Trp–
Phe–Phe–Asn–Tyr–Tyr–X (I)

in der X

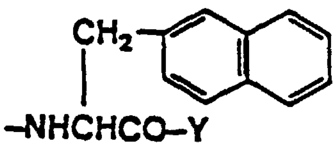

oder

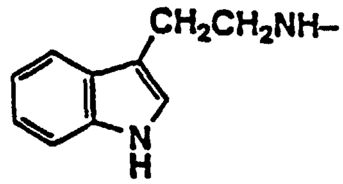

bedeutet (wobei Y eine Hydroxy-, Niederalkoxy, Benzyloxy-, Benzhydryloxyoder Aminogruppe darstellt) oder ein pharmazeutisch verträgliches Salz davon.

2. Peptidverbindung nach Anspruch 1, wobei X

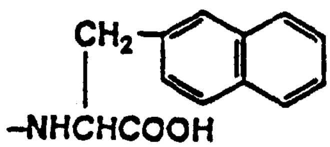

bedeutet oder ein pharmazeutisch verträgliches Salz davon.

3. Peptidverbindung nach Anspruch 1, wobei X

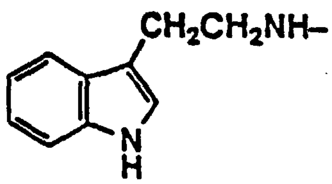

bedeutet oder ein pharmazeutisch verträgliches Salz davon.

4. Das Peptid nach einem der Ansprüche 1 bis 3, welches als Endothelinrezeptor-hemmender Wirkstoff verwendet wird.

5. Die Verwendung des Peptids nach einem der Ansprüche 1 bis 3 zur Herstellung eines Arzneimittels zur Behandlung von Hypertonie, Asthma, Schlaganfall, Angina pectoris, akutem Nierenversagen, Herzinfarkt und cerebralen Vasospasmus.

**Revendications**

1. Peptide représenté par la formule (I) suivante

**Gly-Asn-Trp-His-Gly-Thr-Ala-Pro-Asp-Trp-Phe-Phe-Asn-Tyr-Tyr-X** (I)

dans laquelle X représente un reste

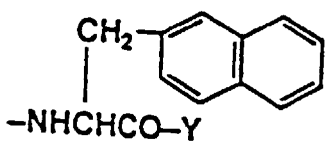 ou 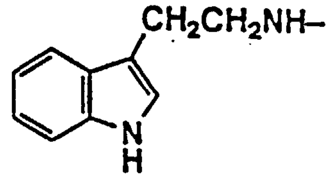

où Y représente un groupe hydroxy, alcoxy inférieur, benzyloxy, benzhydryloxy ou amino, ou sel d'un tel peptide, admissible en pharmacie.

**2.** Peptide conforme à la revendication 1, dans lequel X représente

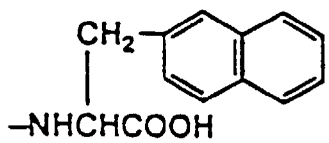

ou sel d'un tel peptide, admissible en pharmacie.

**3.** Peptide conforme à la revendication 1, dans lequel X représente

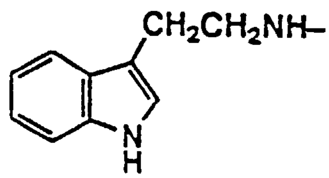

ou sel d'un tel peptide, admissible en pharmacie.

**4.** Peptide conforme à l'une des revendications 1 à 3, destiné à être utilisé comme agent antagoniste des récepteurs de l'endothéline.

**5.** Emploi d'un peptide conforme à l'une des revendications 1 à 3 pour la préparation d'une composition pharmaceutique destinée au traitement de l'hypertension, de l'asthme, de l'apoplexie cérébrale, de l'angine de poitrine, de l'insuffisance rénale aiguë, de l'infarctus du myocarde ou de l'angiospasme cérébral.